# EUROPEAN PATENT SPECIFICATION

(11) **EP 4 398 981 B1**
(45) Date of publication and mention of the grant of the patent: **11.06.2025**
(21) Application number: 22769310.8
(22) Date of filing: 02.09.2022
(51) Int. Cl.: A61N 1/372

(54) **A DELIVERY SYSTEM FOR IMPLANTING A MEDICAL DEVICE**
LIEFERSYSTEM ZUR IMPLANTATION EINES MEDIZINPRODUKTS
SYSTÈME DE LIVRAISON POUR L'IMPLANTATION D'UN DISPOSITIF MÉDICAL

(30) Priority: 10.09.2021 EP 21195912
(43) Date of publication of application: 17.07.2024
(73) Proprietor: BIOTRONIK SE & Co. KG, 12359 Berlin (DE)
(72) Inventor: VAN OOYEN, André, 10717 Berlin (DE); JACOB, Denny, 12277 Berlin (DE); MEHLICH, Andreas, 10318 Berlin (DE); SIEGEL, Philipp Konstantin, 10318 Berlin (DE)
(74) Representative: Biotronik Corporate Services SE
(86) International application number: PCT/EP2022/074493
(87) International publication number: WO 2023/036709

(56) References cited:
- WO-A1-2020/043481
- US-A1- 2015 273 207
- US-A1- 2016 243 355

## Description

The invention is directed to a delivery system for implanting a medical device within a patient's body. Such a medical device may be a pacemaker, for example a leadless pacemaker. The invention is further directed to an assembly comprising a delivery system and to a method for releasing a medical device from a delivery system.

Delivery systems for implanting medical devices, such as leadless pacemaker, into a patient's body, typically comprise a delivery catheter, wherein the medical device is connected to said catheter in order to guide said medical device to an implant location. During implantation, once the medical device reaches the implant location, the medical device is detached from the delivery catheter, wherein the medical device remains at the implant location, and wherein the delivery catheter is removed from the patient's body.

When using such a delivery system for implanting a medical device, it must be ensured that the medical device is firmly connected to the delivery catheter during the implantation procedure until the medical device reaches the implant location, since an unexpected detaching of the medical device may cause complications. In addition, the delivery system must ensure that the medical device can be released from the delivery system at the target location, i.e. the implant location. However, some medical devices require testing prior to releasing in order to assure the correct functioning of said medical devices.

Such tests may be so-called tug tests and/or electrical tests, wherein a tug test is performed by exerting a pulling force to the medical device in order to check whether the medical device is sufficiently secured to the tissue of the patient's body. An electrical test provides information as to whether a medical treatment using an electromagnetic field, for example a proper pacing operation and electrical signal reception of a leadless pacemaker, is performed in a desired manner. For such tests, the medical device needs to be at least partially detached from the delivery system in order to give the medical device free room of motion. A release of the medical device from the delivery system should take place only if the testing is successfully completed. Otherwise, if the test was not successful, it must be ensured that the medical device can be repositioned or even completely removed.

A delivery system suitable for implantation according to the description above is disclosed in document WO 2020/043481 A1. The known system comprises an adapter piece moveable with a mandrel within a delivery catheter of the delivery system, wherein the adapter piece is used to secure a medical device via a tethering member to said delivery system in a first position in which the adapter piece is located inside the delivery catheter and to release the medical device in a second position in which the adapter piece is located outside the delivery catheter. The known delivery system is suitable for implantation of a medical device but especially the adapter piece and therefore the entire delivery system has several drawbacks. The adapter piece of the known system has a complex design so that the system is not suitable for automated manufacturing and assembling. Additionally, the connection of the mandrel as well as the connection of the tethering member offset from the centre of the adapter piece can lead to unfavourable tilting of the adapter piece both when pulling and pushing the mandrel. A further disadvantage of the known adapter piece is that, due to its shape and dimensions, comparatively large frictional forces occur if the adapter piece is moved within the delivery catheter which may interfere with the tug test.

US 2015/0273207 discloses a tether subassembly, which may be employed by a tool that deploys an implantable medical device, includes a test segment for verification of adequate fixation of the device at an implant site.

US 2016/0243355 discloses a tethering member which extends within a lumen of a shaft, which may be part of a catheter assembly that further includes a locking member extending within a locking lumen and having a distal tip located in proximity to an aperture of the shaft.

Therefore, it is an object of the present invention to provide a delivery system and assembly which are improved with regard to above drawbacks of the prior art. Further, it is an object of the present invention to specify an easy and reliable method for releasing a medical device from such delivery system.

The above problem is solved by a delivery system according to claim 1, an assembly according to claim 13, and a not claimed method for releasing a medical device from a delivery system.

In particular, the problem is solved by a delivery system for implanting a medical device comprising a delivery catheter, an adapter piece, a mandrel, and a tethering member. The delivery catheter comprises an inner lumen, wherein the mandrel is received in the inner lumen of the delivery catheter and movable with respect to the delivery catheter. Further, the tethering member comprises a first end, a second end and a positive-locking member fixed to the second end. Furthermore, the adapter piece has a longitudinal axis and comprises a proximal end face and a lateral surface, wherein the adapter piece forms a fixed connection between a distal end of the mandrel and the first end of the tethering member. By moving the mandrel with respect to the delivery catheter, the adapter piece is displaceable between a first position within the inner lumen of the delivery catheter and a second position in which the adapter piece is arranged outside of the inner lumen of the delivery catheter. In the first position of the adapter piece the second end of the tethering member runs along the lateral surface of the adapter piece substantially parallel, i.e. parallel or at a small angle (less than 20 °), to the longitudinal axis and beyond the proximal end face of the adapter piece such that the positive-locking member adjoins the proximal end face thereby providing a locked connection with the adapter piece. In the first position, the adapter piece is located at least partly within the inner lumen of the delivery catheter.

The delivery system according to the invention comprises the delivery catheter located proximally with respect to an implantable medical device, wherein during a surgery the delivery catheter is directed towards a health care practitioner (HCP). A distal end of the delivery catheter, which may be provided by an end cap, is connected to the medical device as long as the adapter piece is in its first position. To be guided for example through the superior vena cava into a patient's heart to access, via the right atrium, the right ventricle, the delivery catheter may comprise a flexible material.

The mandrel, the adapter piece, the tethering member and its positive-locking member may form a pushing catheter located within the inner lumen of the delivery catheter.

The mandrel is located proximally from the adapter piece, extends to the proximal end of the delivery catheter and is fixed with its distal end to the adapter piece. The mandrel may be formed by a stiff but bendable wire having, for example, a circular or angular cross section, and pushes or pulls the adapter piece and thereby the medical device coupled to the tethering member in the desired position. The mandrel may be pushed or pulled manually or automatically at a handle provided at the proximal end of the delivery catheter.

The adapter piece serves to control the establishment and release of the connection of the medical device with the delivery system and provides the connection until release. The position of the adapter piece with regard to the delivery catheter is critical for its controlling function. The position of the adapter piece is adjusted by pushing or pulling the mandrel, which is fixedly connected to the adapter piece. The proximal end face of the adapter piece runs substantially perpendicular or inclined to its longitudinal axis. The proximal end face may be formed by a flat or curved surface. The lateral surface runs at least partly or fully parallel to the longitudinal axis and between the proximal end face and a distal end face of the adapter piece. The distal end face is located opposite the proximal end face alpng the longitudinal axis of the adapter piece. The lateral surface forms the outer surface in between the proximal end face and the distal end face of the adapter piece. For a, for example, substantially cylindrical shaped adapter piece, the lateral surface is located between the proximal end face and the distal end face of the adapter piece, wherein the cylindrical lateral surface is provided radially offset from the longitudinal axis of the adapter piece. The length of the adapter piece is its extension in the direction parallel to its longitudinal axis or, in other words, its dimension between the proximal end face and the distal end face.

In a connection state, i.e. in the first position of the adapter piece with regard to the delivery catheter, the tethering member located at the distal end of the adapter piece forms a loop that extends through an opening of a connection member of the medical device so that the medical device is coupled to the delivery system. Accordingly, the tethering member is a flexible member configured to form the required loop. The loop starts at the distal end of the adapter piece where the first end of the tethering member is fixed to the adapter piece and ends at the positive locking member (i.e. at the second end of the tethering member). In the connection state, the proximal end face of the adapter piece and the positive-locking member form a positive / interlocking connection, wherein the proximal end face of the adapter piece and inner surface of the delivery catheter hinder the movement of the positive-locking member into distal direction with regard to the adapter piece. In other words, the intermediary space between the lateral surface of the adapter piece and the inner surface of the delivery catheter is so small that the positive locking member is trapped at the proximal end face of the adapter piece. Accordingly, the outer diameter of the positive-locking member is greater than the intermediary distance of the lateral surface of the adapter piece and the inner surface of the delivery catheter. However, this intermediary distance is - at least at a part of the circumference of the adapter piece - chosen such, that the tethering member in a section different from the positive-locking member may run along the lateral surface of the adapter piece and along the inner surface of the delivery catheter substantially parallel to the longitudinal axis of the adapter piece. In the second position of the adapter piece, in which the adapter piece is arranged outside of the inner lumen of the delivery catheter the inner surface of the delivery catheter does not form any limiting surface any more with regard to the distal movement of the positive-locking member and the second end of the tethering member with the positive-locking member may move freely with regard to the adapter piece thereby opening the loop and allowing release of the medical device from the delivery system. In the second position, the second end of the tethering member is no longer clamped or jammed between the inner surface of the delivery catheter and the lateral surface of the adapter piece, so that the positive locking connection between the second end of the tethering member and the adapter piece is released. Accordingly, the connection between the adapter piece and the second end of the tethering member is therefore in generally released by a simple uniaxial movement of the adapter piece from the first into the second position, i.e. by pushing the mandrel in the distal direction. On the other hand, a similar uniaxial movement is also sufficient to connect the second end of the tethering member to the adapter piece during manufacturing or in preparation of a surgery, for example, by pulling the mandrel and thereby the adapter piece in the proximal direction to arrive at the first position.

As the positive locking of the positive-locking member is provided by the proximal end face of the adapter piece, no additional, specific structure for positive locking is required at the lateral surface of the adapter piece. Accordingly, a simple design / form may be chosen for the adapter piece. Examples for such design are described below in detail. The simple design allows cost-efficient automated manufacturing and automated assembling, for example by turning, milling and/or drilling.

The accommodation of the positive-locking member at the proximal end of the adapter piece further allows a construction of the adapter piece such that friction surfaces at the adapter piece and the distal end of the tethering member interacting with the inner surface of the delivery catheter are minimized such that interference with a tug test is reduced. Further, the connection of the adapter piece to the mandrel and to the first end of the tethering member may be provided more centrally than in the prior art so that the whole adapter piece moves better centred within the delivery catheter.

Once the medical device has reached its implant location, the coupling between the delivery catheter and the medical device may be released by moving, e.g. pushing, the adapter piece from its first into its second position as explained above. However, if a test such as a tug test and/or electrical test shall be performed prior to the full release, the adapter piece may also be displaced into a so-called intermediate position so that the medical device is released from the end cap but the adapter piece still remains inside the inner lumen of the delivery catheter. In this (first) position of the adapter piece, the coupling of the medical device with the delivery catheter via the tethering member still remains in effect by positive locking.

A tug test as well as an electrical test may be performed in the intermediate position. The tug test checks proper fixation of the medical device on tissue by means of anchoring members at the target location by pulling on the medical device via the delivery system into the proximal direction using, for example, the mandrel. Further, an electrical testing may assure a proper functioning of electrical stimulation by electrodes located at the medical device. If during the testing it is found that the medical device has to be relocated, the adapter piece may again be retracted into the delivery catheter by pulling the mandrel and together with the mandrel the adapter piece in the proximal direction such that the medical device again comes into engagement with the end cap of the delivery catheter. The medical device may then be repositioned at a new implant location. In one embodiment, the adapter piece may only be moved into the second position, i.e. the medical device may only be released from the delivery system after a successful testing.

During manufacturing the distal end of the mandrel is fixedly connected to the adapter piece, for example by welding. Further, the first end of the tethering member is fixedly connected to the adapter piece, for example also by welding or by crimping as described below in detail. Therefore, the adapter piece forms a fixed connection between the distal end of the mandrel and the first end of the tethering member. In one embodiment a borehole may be provided on the proximal end face of the adapter piece and/or at the distal end of the adapter piece to receive the mandrel and/or the first end of the tethering member. Alternatively or additionally, the distal end of the mandrel and/or the first end of the tethering member may be directly connected to an end face of the adapter piece. Regardless of the type of connection between the adapter piece and the mandrel or the first end of the tethering member, a centred connection, particularly a centred connection of the mandrel, prevents tilting of the adapter piece when pushing and/or pulling forces are applied to the adapter piece via the mandrel.

After the connection of the distal end of the mandrel and the first end of the tethering member to the adapter piece, the tethering member may be connected to the medical device, i.e. to a connection member of the medical device.

In one embodiment, the adapter piece is rotationally symmetric with respect to its longitudinal axis. In the context of the invention, rotational symmetry means that the adapter piece has an axis of rotation, i.e. the longitudinal axis, wherein rotation about an angle around said axis, which is different to 360°, causes the rotated body to coincide with the body in the starting position. Due to the rotationally symmetrical design of the adapter piece, manufacturing is considerably simplified. In this way, the symmetrical design enables, for instance, manufacturing with common machining processes such as turning and milling. An exemplary adapter piece may be a rotationally symmetrical cylinder, provided optionally with rounded edges, chamfered edges, or broken edges. Further, due to the symmetrical design of the adapter piece, the friction surfaces between the lateral surface of the adapter piece and the inner surface of the inner lumen of the delivery catheter are evenly arranged when viewed in the circumferential direction of the adapter piece. This allows for even distribution of friction forces between the adapter piece and the delivery catheter if the adapter piece is located in the inner lumen of the delivery catheter, preventing, for example, an unfavourable tilting of the adapter piece.

In one embodiment, a radial distance between the longitudinal axis and the lateral surface of the adapter piece varies along the circumferential direction of the adapter piece, wherein this variation runs along at least part of the length of the adapter piece or the full length of the adapter piece. In one embodiment the adapter piece may comprise a single groove or the like at its lateral surface running in longitudinal direction in which the tethering member may be placed in the first position of the adapter piece. In another embodiment the adapter piece may comprise a plurality of longitudinally running ribs and grooves or notches at its lateral surface, for example with curved edges, forming a substantially star-shaped design on its cross section. This design can still cost-efficiently manufactured and assembled and provides a well-defined location for the tethering member in the first position of the adapter piece.

In one aspect, the surface of the inner lumen (i.e. the inner surface) of the delivery catheter and the adapter piece comprise one to six contact lines or surfaces. For example, a cylindrically shaped adapter piece comprises one contact surface with the inner surface of the delivery catheter. In another embodiment, an adapter piece in the above described star shape on its cross section, in which grooves or notches extend parallel to the longitudinal axis of the adapter piece over the entire lateral surface, may comprise, for example, two to six contact surfaces or contact lines, wherein each contact line represents the contact between the longitudinally extending ribs of the adapter piece and the inner surface of the delivery catheter. The number of ribs in such a design is therefore decisive for the number of angles of rotation defining the rotational symmetry.

The friction forces between two components are affected in particular by the size of the friction surfaces, i.e. the contact lines or surfaces between the adapter piece and the inner surface of the delivery catheter. In this embodiment, a reduction of the friction surface is achieved by an appropriate design, for example the star-shaped design. Alternatively or additionally, the dimensions of the adapter piece may be reduced. The overall length of the adapter piece may be between 3 mm to 4 mm, preferably between 3 mm to 3.4 mm. Further, a diameter or maximum in-plane distance between two points of the lateral surface of the adapter piece may be between 1 mm to 2 mm, preferably between 1.1 mm to 1.6 mm. Further, the adapter piece may, once it is arranged inside the inner lumen of the delivery catheter, contact the inner surface of the delivery catheter over part of its longitudinal extent. The aforementioned measures cause an increased trackability of the pushing catheter within the delivery catheter.

In one embodiment, the adapter piece comprises a through-hole extending along or parallel to its longitudinal axis configured to receive the distal end of the mandrel and/or the first end of the tethering member. The mandrel and/or the first end of the tethering member may be fixed within the through-hole of the adapter piece forming a permanent connection, for example by welding or gluing. The mandrel which is initially partially received and fixed inside the through-hole (for example by welding) may serve as a stop surface for a subsequently inserted first end of the tethering member. The arrangement of the through-hole along the longitudinal axis ensures the previously mentioned advantageous centred fastening, in particular of the distal end of the mandrel, which results in a push and/or pull force acting on the adapter piece in a centred manner and thus prevents tilting. For the manufacturing of the filigree adapter piece, the use of through-holes in automated manufacturing is significantly easier and less labour-intensive, since chips that may otherwise accumulate in the borehole do not have to be removed. In another embodiment, the through-hole may also be angled with respect to the longitudinal axis.

In one embodiment, the through-hole comprises an abutting face extending vertically to the longitudinal axis. An abutting face may be manufactured in particular by first drilling a through-hole with a smaller diameter and then drilling a borehole with a greater diameter at the position of the smaller through-hole thereby forming a first section of the through-hole having a greater diameter and a second section having a smaller diameter. The abutting face separates the first and the second section from each other. The abutting face formed in this way may help to predefine the insertion depth of the mandrel and/or the tethering member in the adapter piece. In addition, the use of different borehole diameters for different diameters of the mandrel and the first end of the tethering member may provide for simplified fastening of the mandrel and/or of the first end of the tethering member to the adapter piece. In one embodiment the first section of the through-hole having the greater diameter is located at the proximal end of the through-hole and is adapted to receive the distal end of the mandrel.

In one aspect, the adapter piece comprises a tubular section at its distal end, wherein the tubular section is configured to receive the first end of the tethering member, and wherein the tubular section has a smaller outer diameter than a proximal section of the adapter piece. The outer diameter of the tubular section may therefore be between 0.3 mm to 0.8 mm, preferably between 0.5 mm to 0.7 mm. The longitudinal length of the tubular section may be between 1.3 mm to 2 mm, preferably between 1.5 mm to 1.7 mm, wherein the proximal section may comprise the aforementioned diameter or maximum in-plane distance. Further, the tubular section may comprise a borehole or a through-hole in order to receive the first end of the tethering member. The through-hole may also be configured to receive a portion of the mandrel at the proximal section of the adapter piece. Furthermore, the distal tubular section may be configured to be crimped so that the first end of the tethering member, when received inside the tubular section, may be connected to the adapter piece by crimping. This is, in particular, an advantageous, reliable fixing method for a multi-stranded tethering member. The adapter piece may be a multi-piece component but is alternatively a one-piece component.

As already indicated above, in one embodiment, the lateral surface of the adapter piece comprises at least one groove or curved notch extending parallel to the longitudinal axis. The groove or curved notch may be configured to receive the second end of the tethering member, so that the second end of the tethering member runs along said groove or notch while the adapter piece is in its first position. The adapter piece may also comprise a plurality of such curved notches in between the proximal end face and the distal end face thereby forming a cross-section having a star shape. These grooves and notches may guide air and/or a flushing/rinsing fluid and/or radiopaque fluid from proximal end to the distal end of the delivery system or into the opposite direction.

In one aspect, the positive-locking member of the tethering member has a trapezoidal, a teardrop or a spherical shape. Regardless of the shape of the positive-locking member, the positive-locking member is always configured to adjoin the proximal end face of the adapter piece, if the adapter piece is in its first position. The positive-locking member may be formed integrally with the tethering member or form a separate element fixed thereto. The shape of the positive-locking member may be chosen such that it best fits the shape of the adapter piece and provides the best and most reliable form-locking connection in the first position of the adapter piece.

As indicated above, in one embodiment, the tethering member forms a loop while the adapter piece is in its first position. This may be achieved by passing the tethering member, which is attached to the adapter piece with its first end, through an opening of a connection member of the medical device, wherein the tethering member then extends back towards the distal end of the adapter piece, wherein the second end of the tethering member runs along the lateral surface of the adapter piece thereby forming a loop. The medical device is thus located at one end of the loop, with the adapter piece located at the opposite end. Displacing the adapter piece from its first position into its second position releases the positive-locking connection of the second end of the tethering member with the adapter piece and thereby opens the loop.

Further, the tethering member may be formed for example by a cable, suture, or wire and may be one-stranded or multiple-stranded. The tethering member may be generally flexible and adapted to form a loop for connecting the medical device to the delivery system. Additionally, the tethering member may exhibit a substantial rigidity (while allowing a sufficient bendability).

In one embodiment, as described above, the dimensions of the inner lumen of the delivery catheter, the adapter piece and the positive-locking member are chosen such that the positive-locking member is blocked from disengaging from the adapter piece in the first position but enabled to disengage from the adapter piece in the second position, when the adapter piece is fully moved out of the inner lumen of the delivery catheter.

In one embodiment, the mandrel and/or the adapter piece and/or the tethering member and/or the positive-locking member comprises or consist of a biocompatible polymer or metal material, for example stainless steel. Further, the adapter piece may comprise materials which provide a good trackability. Thus, a coating may be provided at the adapter piece and/or the tethering member and/or the inner surface of the delivery catheter to lower the friction of the respective components. Further, the tethering member and/or the adapter piece may comprise a radiopaque material such that the tethering member may be visualized for example in an X-ray examination.

Furthermore the delivery system may comprise a stopper which is arranged on the mandrel proximal to the adapter piece. The stopper could be made of the same material as the adapter piece and could be fixed to the mandrel in the same way as the adapter piece (welded, glued, pressed onto or any other suitable way). The stopper ensures in this embodiment that the positive locking member and the tethering member are not accidentally pushed back into the inner lumen of the delivery catheter, if the mandrel is pushed in the distal direction.

The above problem is also solved by an assembly comprising a delivery system according to the preceding description and a medical device comprising a connection member, wherein the tethering member is connected to the connection member while the adapter piece is in the first position.

In one embodiment, the connection member comprises an opening through which the loop-shaped tethering member extends while the adapter piece is in the first position. Hence, the medical device is fixedly connected to the delivery system if and while the adapter piece is in its first position. The connection member of the medical device may be connected to a housing component of the medical device, wherein alternative shapes of the connection member may be provided. However, independent of the shape of the connection member, using round and/or curved contours or at least contours without edges may help to ensure that the positive-locking member does not get caught if the positive-locking member is released from the adapter piece. Further, using contours without edges ensure that the tethering member does not rip. Thus, if the positive-locking member is released from the adapter piece the tethering member may be pulled out of engagement from the connection member, in particular by passing the positive-locking member through the opening formed by the connection member, such that the medical device is released from the delivery system. Accordingly, in this embodiment, the opening of the connection member is greater than the outer diameter of the positive-locking member in order to allow passing of the positive-locking member through the opening.

Further, the above problem is solved by a method for releasing a medical device from a delivery system, comprising:
- providing the delivery system in a state in which the medical device is coupled to the delivery system, the delivery system comprising a delivery catheter having an inner lumen, a mandrel received in the inner lumen of the delivery catheter and movable with respect to the delivery catheter, a tethering member comprising a first end, a second end and a positive locking member fixed to the second end, an adapter piece having a longitudinal axis and comprising a proximal end face and a lateral surface, wherein the adapter piece forms a fixed connection between a distal end of the mandrel and the first end of the tethering member, wherein the adapter piece is, by moving the mandrel with respect to the delivery catheter, located in a first position within the inner lumen of the delivery catheter, wherein in the first position of the adapter piece the second end of the tethering member runs along the lateral surface of the adapter piece parallel to the longitudinal axis and beyond the proximal end face of the adapter piece such that the positive-locking member adjoins the proximal end face thereby providing a locked connection with the adapter piece and forming a loop for connection with the medical device, and
- enabling disengagement of the positive-locking member from the adapter piece by displacing the adapter piece from the first position into a second position in which the adapter piece is arranged outside of the inner lumen of the delivery catheter by moving the mandrel. Thereby disconnection of the medical device from the tethering member is enabled, too.

The above method and system may be used for medical devices such as implantable leadless pacemaker (ILP) or any other kind of implantable sensor device suitable for implantation within a chamber of the heart. Such sensors could measure pressure, potassium concentration and/or oxygen saturation.

Prior to the disengagement of the positive-locking member from the adapter piece, the medical device is moved to and placed at an implant target location, for example on a tissue of the patient's body, such as a myocardial tissue by partial releasing the medical device from the delivery system. The medical device may comprise anchoring members, which may have the shape of tines, wires, sheets or tubes, extending from the medical device and are used to fix the medical device to the tissue at this location. A tug test and/or an electrical test may be performed subsequently to the fixing of the medical device. If the test(s) is/are successful, the HCP may release the medical device by displacing the adapter piece from its first into its second position. However, if the test is unsuccessful, the HCP may relocate the medical device using the delivery system. In this case, the medical device remains connected to the delivery system until the medical device has reached an appropriate implantation location and passed testing, if required.

The present invention will now be described in further detail with reference to the accompanying schematic drawings, wherein
- Fig. 1: shows a cross section of the human heart with an implanted medical device and a delivery system,
- Fig. 2: shows a side view of a medical device to be implanted in a patient's heart,
- Fig. 3: shows a side view of a first embodiment of a delivery system for implanting a medical device coupled to a medical device,
- Fig. 4: shows a closed-up side view of the medical device according to Fig. 3 with a connection member through which a loop-shaped tethering member extends,
- Fig. 5A: shows a side view of a first embodiment of an adapter piece which may be used in the delivery system according to Fig. 3,
- Fig. 5B: shows a front view of the proximal end face of the adapter piece of Fig. 5A,
- Fig. 5C: shows a perspective view of the partly cut-out representation of a delivery catheter of the delivery system of Fig. 3 with an adapter piece according to Fig. 5A and a positive-locking member in a first position,
- Fig. 6A: shows a side view of a second embodiment of an adapter piece which may be used in the delivery system according to Fig. 3,
- Fig. 6B: shows a front view of a proximal end face of the adapter piece of Fig. 6A,
- Fig. 6C: shows a perspective view of the partly cut-out representation of a delivery catheter of the delivery system of Fig. 3 with an adapter piece according to Fig. 6A and a positive-locking member in a first position,
- Fig. 7A: shows a side view of a third embodiment of an adapter piece which may be used in the delivery system according to Fig. 3,
- Fig. 7B: shows a cross section of the adapter piece of Fig. 7A along axis C-C shown in Fig. 7A,
- Fig. 7C: shows a front view of a distal end face of the adapter piece of Fig. 7A,
- Fig. 7D: shows a longitudinal section of the adapter piece of Fig. 7A along axis D-D shown in Fig. 7C, and
- Fig. 7E: shows a longitudinal section of the adapter piece of Fig. 7A along axis A-A shown in Fig. 7C.

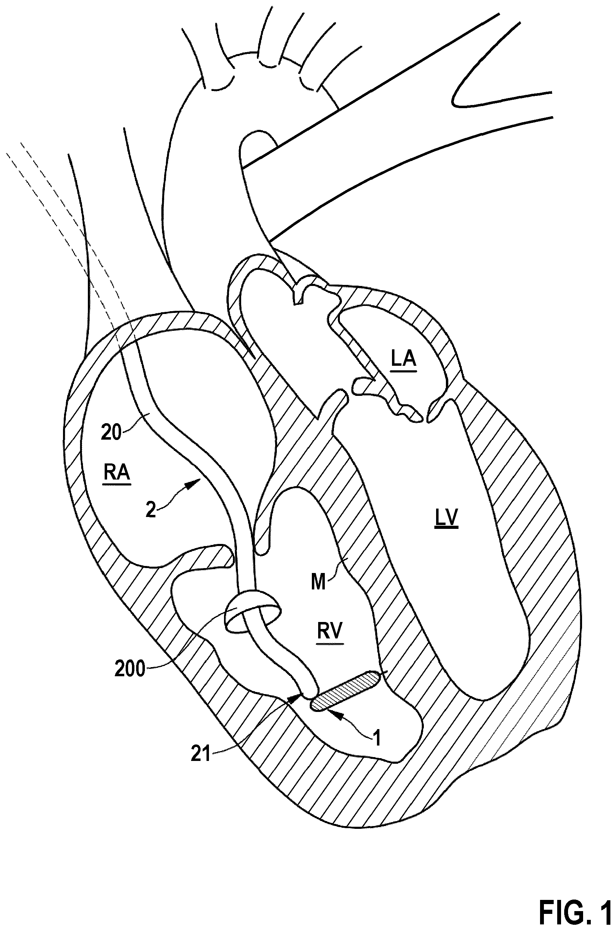

Fig. 1 depicts a human heart comprising a right atrium RA, a right ventricle RV, a left atrium LA and a left ventricle LV, a medical device 1 being implanted into the right ventricle RV, the medical device 1, for example, being a leadless pacemaker device for providing a pacing of the heart's activity at the right ventricle RV.

For implantation the medical device 1 is placed by means of a delivery system 2 at an implant location, for example in the right ventricle RV - as illustrated in Fig. 1 - such that it comes to rest on myocardial tissue M and fixed there using its anchoring members 12.

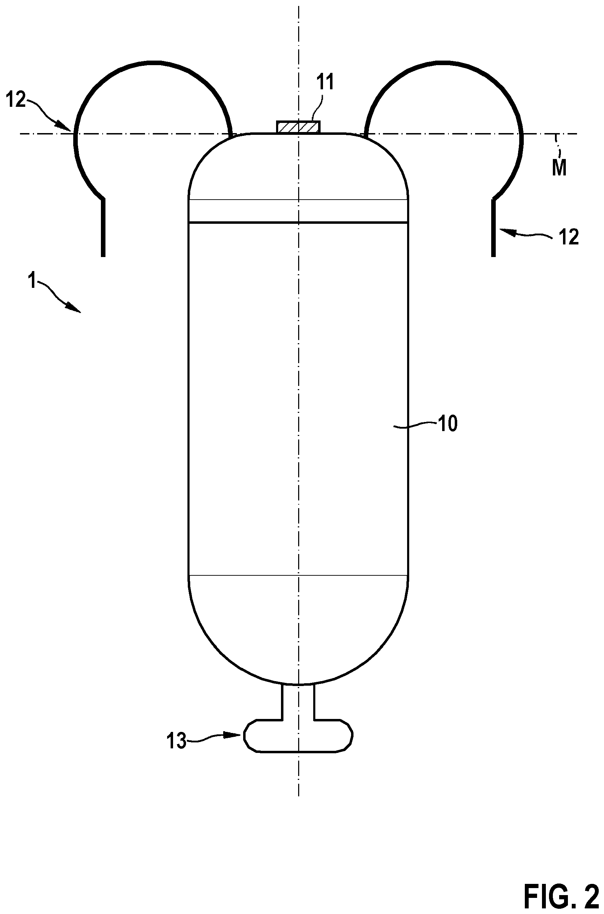

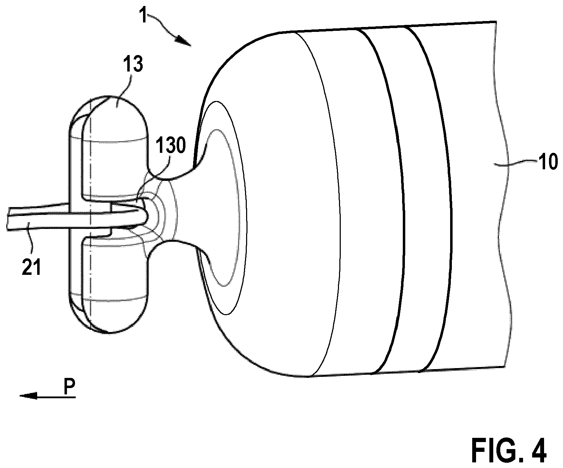

Fig. 2 shows an example of a medical device 1 being a leadless pacemaker device with a housing 10, wherein the medical device 1 comprises a plurarity of anchoring members 12 in the shape of tines extending from the housing 10 of the medical device 1 to fix the medical device to myocardial tissue M of the patient. Further, the medical device 1 comprises an electrode 11 at its distal end which serves for pacing and cardiac stimulation. A second electrode may be formed by a proximal end of the housing. To guide the medical device 1 to a target location the medical device 1 is connected to the delivery system 2 by a tethering member 21 which is released once the medical device 1 is mechanically fixed to the tissue of the patient. However, before releasing the medical device usually a testing such as a tug testing and/or electrical testing, is performed in order to ensure secure mechanical fixing as well as the correct functioning of the medical device 1. The medical device 1 comprises a connection member 13, which is used to releasably connect the medical device 1 via a tethering member 21 to the delivery system 2. An embodiment of the connection member 13 is shown in more detail in Fig. 4.

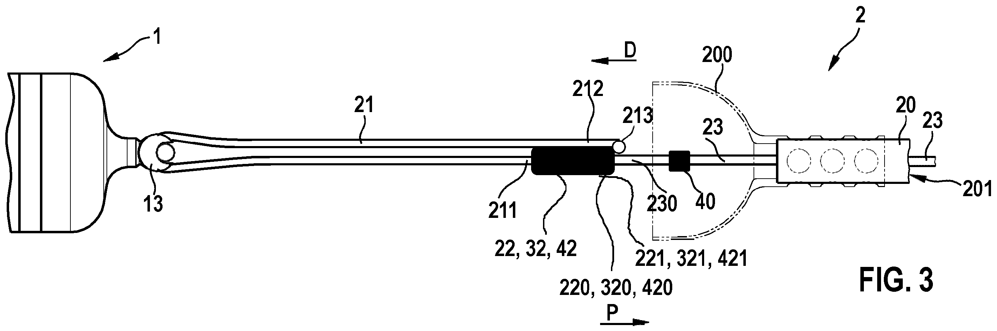

The delivery system shown in Fig. 3 comprises a delivery catheter 20 having an inner lumen 201 and a pushing catheter formed by a mandrel 23, an adapter piece 22 and a tethering member 21 with a positive-locking member 213.

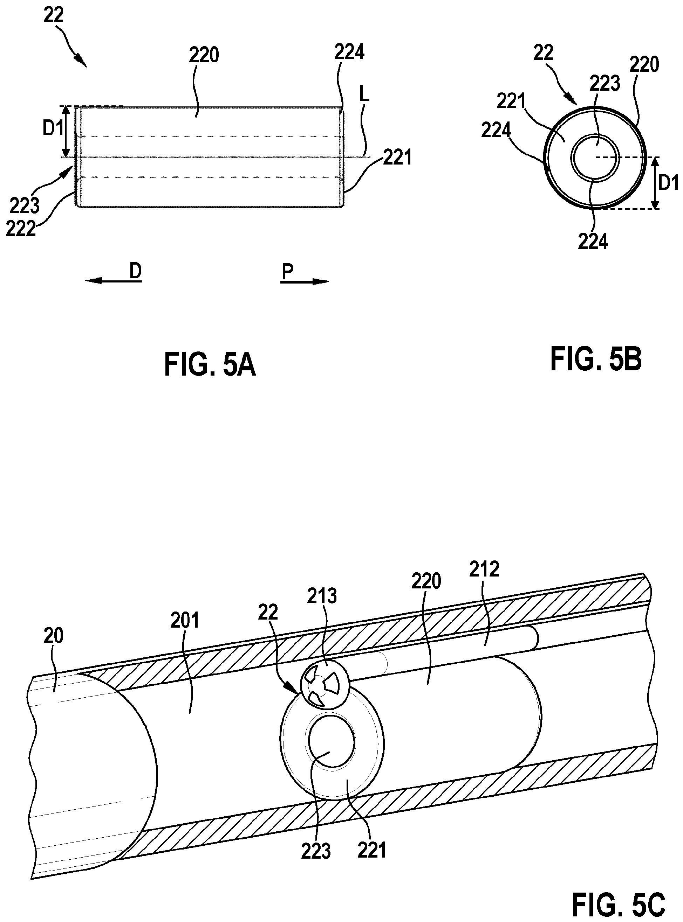

The tethering member 21 of delivery system 2, as shown in Fig. 3, comprises a first end 211 which is fixedly connected to an adapter piece 22, for example by welding, and a second end 212 comprising the positive-locking member 213. The positive-locking member 213 shown in Fig. 3 has a spherical shape. Regardless of the shape, the positive-locking member 213 needs always to be configured to provide a positive locking with the adapter piece 22, while the adapter piece 22 is in its first position. This is achieved, inter alia, by the adapter piece 22 being located within the inner lumen 201 of the delivery catheter 20, with the second end 212 of the tethering member 21 extending along the lateral surface 220 of the adapter piece 22 and the positive-locking member 213 adjoining the proximal end face 221 of the adapter piece 22, wherein the positive-locking member 213 and therefore the second end 212 of the tethering member 21 cannot be moved in the distal direction D without moving the adapter piece 22 which is however connected to a mandrel 23, for example by welding. The first position of adapter piece 22 is shown in Fig. 5C.

Even though the adapter piece 22 shown in Fig. 3 is depicted in its second position, the second end 212 of the tethering member 21 is still running along the lateral surface 220 of the adapter piece 22 parallel to a longitudinal axis L (see Fig. 5A) and beyond a proximal end face 221 of the adapter piece 22, wherein the positive-locking member 213 adjoins the proximal end face 221 of the adapter piece 22. However, the tethering member 21 shown in Fig. 3, wherein the adapter piece 22 is in its second position does not provide a locked connection with the adapter piece 22 and may therefore be released at any second. Embodiments of the adapter piece 22 will be described in more detail with respect to FIGs. 5A to 7E. It is easily seen in Fig. 3 that release of the positive-locking member 213 from the lateral surface of the adapter piece 22 is not hindered by the delivery catheter 20. Accordingly, in the second position, the medical device 1 can be decoupled from the delivery system as described below in detail.

Fig. 3 further shows two arrows D and P pointing in the distal direction D and the proximal direction P of the delivery system. The proximal end face 221 of the adapter piece 22 is directed in the proximal direction P. A distal end face is located at the distal end (opposite of the proximal end face 221) of the adapter piece 22.

The delivery system 2 further comprises the proximally located delivery catheter 20 comprising an end cap 200 and an inner lumen 201. The mandrel 23 running through the inner lumen 201 of the delivery catheter 20 is connected with its distal end 230 to the adapter piece 22. The mandrel 23 comprises a biocompatible polymer or metal material. Usually the mandrel 23 has a greater or at least equal diameter compared to the tethering member 21. The tethering member 21 may be formed by a cable, suture or wire made from biocompatible polymer or metal material.

The mandrel 23 is sufficiently flexible such that it may be deformed together with the delivery catheter 20 when guiding the delivery catheter 20 through body portions of the patient, but at the same time is rigid and kink-proof such that it may be moved with respect to the delivery catheter 20. Further, the mandrel 23 is used to displace the adapter piece 22 by applying a pushing and/or pulling force to the mandrel 23. The first position of the adapter piece 22 in which the positive-locking member 213 provides a positive form locking connection with the adapter piece 22 (see Fig. 5C), differs from the second position shown in Fig. 3 in that in the first position the adapter piece 22 is located at least partly within the inner lumen 201 of the delivery catheter 20.

Furthermore, Fig. 3 depicts the medical device 1 of Fig. 2 shown at one end in the distal direction D of the delivery system 2. The medical device 1 is, however, not part of the delivery system 2 but of an assembly comprising the medical device 1 and the delivery system 2. The tethering member 21 is guided through an opening 130 (see Fig. 4) of the connection member 13 and back to the adapter piece 22 thereby forming a loop. This loop is disconnected from the connection member 13 in order to release the medical device after implantation. Accordingly, the dimension as well as the shape of the opening 130 of the connection member 13 needs to be greater than the dimensions and the shape of the positive-locking member 213 in order to enable safe release of the medical device 1.

While the assembly is guided through the patient's body to a target location for implantation, the adapter piece 22 is in its first position. The mandrel 23 is retracted such that the adapter piece 22 is located fully inside the inner lumen 201 of the delivery catheter 20. The adapter piece 22 is further positioned such to ensure that the medical device 1 is drawn towards the end cap 200 and thereby fixedly connected to the delivery catheter 20. Hence, a reliable coupling of the medical device 1 and the delivery catheter 20 is established. In this state the medical device 1 may be guided by means of the delivery system 2 towards the target location. The delivery system 2 further comprises a stopper 40 arranged at the mandrel 23 proximal to the adapter piece 22.

Once the medical device 1 has reached the target location and all necessary steps have been taken, i.e., for example, the medical device has been fixed by the anchoring members 12 to the tissue M of the patient, the mandrel 23 is operated (i.e. pushed) in order to displace the adapter piece 22 so that the medical device 1 is released from the end cap 200, wherein the adapter piece 22 still remains inside the inner lumen 201 of the delivery catheter 20. Thereby the medical device 1 is spatially removed from the end cap 200 of the delivery catheter 20, while the connection via the tethering member 21 still remains in effect.

In this so-called intermediate position, a tug test as well as an electrical test may be performed. Therefore, by pulling the medical device 1 away from the tissue M via the tethering member 21 of the delivery system 2 a proper fixing of the medical device 1 at the tissue M by means of the anchoring member 12 may be assured as well as a proper functioning of stimulation by electrodes 11. The tug test may, for example, be performed by pulling the mandrel 23 together with the adapter piece 22 into the proximal direction P.

If during test steps it is found that the medical device 1 is not securely fixed at the tissue M, but is released when applying a predefined force during the tug test, or if it is found that the medical device 1 is not correctly positioned for performing its electrical functions (e.g. a contact resistance is higher than a pre-defined value), the medical device 1 may have to be repositioned. For this, the adapter piece 22 may again be retracted into the delivery catheter 20 by pulling the mandrel 23 and together with the mandrel 23 the adapter piece 22 into the proximal direction P such that the medical device 1 again comes to engage with the end cap 200. The medical device 1 may then be positioned at another location, and testing may be repeated until a proper positioning and functioning of the medical device 1 is confirmed.

Once it is found that the medical device 1 is properly implanted and fixed at the tissue M and that it functions correctly, the medical device 1 is to be released from the tethering member 21. For this, the adapter piece 22 is, by pushing the mandrel 23 into the distal direction D, moved fully out of the inner lumen 201 of the delivery catheter 20, as it is shown in Fig. 3. In the second position of the adapter piece 22 the positive-locking member 213 adjoining the proximal end face 221 of the adapter piece 22 is no longer locked, such that the positive-locking member 213 is released from the adapter piece 22 and the loop formed by the tethering member 21 is opened. The stopper 40 ensures that the positive-locking member 213 is not accidentally pushed again into the delivery catheter due to the distal pushing of the mandrel 23.

The release of the tethering member 21 and thus of the medical device 1 is then, for example, performed by pulling the tethering member 21 into its proximal direction P wherein the positive-locking member 213 slides through the opening 130 of the connection member 13. When the positive-locking member 213 is released from the adapter piece 22, the tethering member 21 and the positive-locking member 213 of the second end 212, respectively, are pulled through the opening 130.

Alternative shapes and dimensions of a connection member 13 may be used in an assembly with a differently shaped positive-locking member 213. However, independent of the shape of the connection member, using round and/or curved contours or at least contours without edges help to ensure that the positive-locking member 213 does not get caught when the positive-locking member 213 is released from the adapter piece 22 and pulled through the opening 130 of the connection member 13 of the medical device 1.

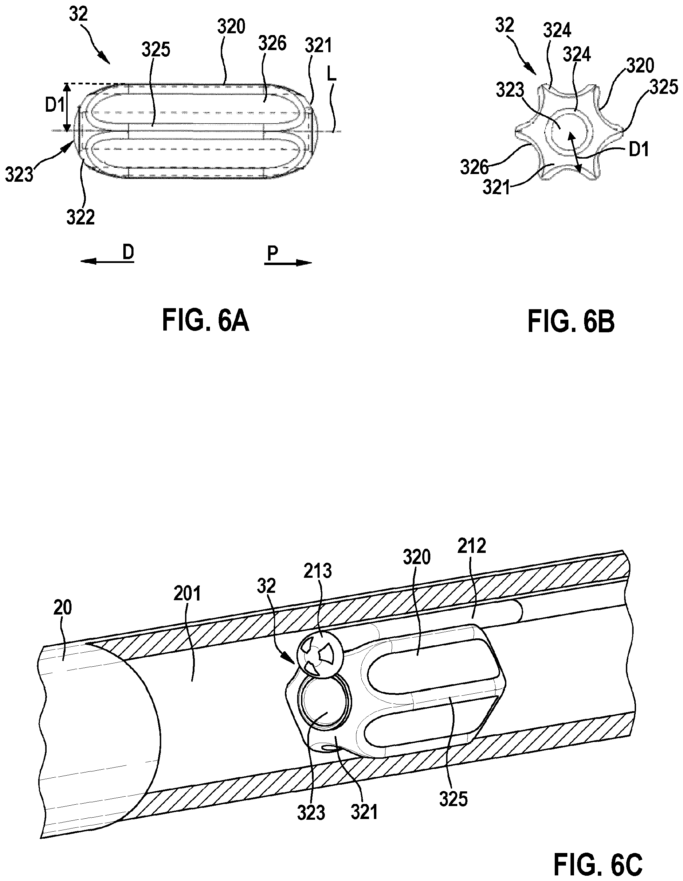

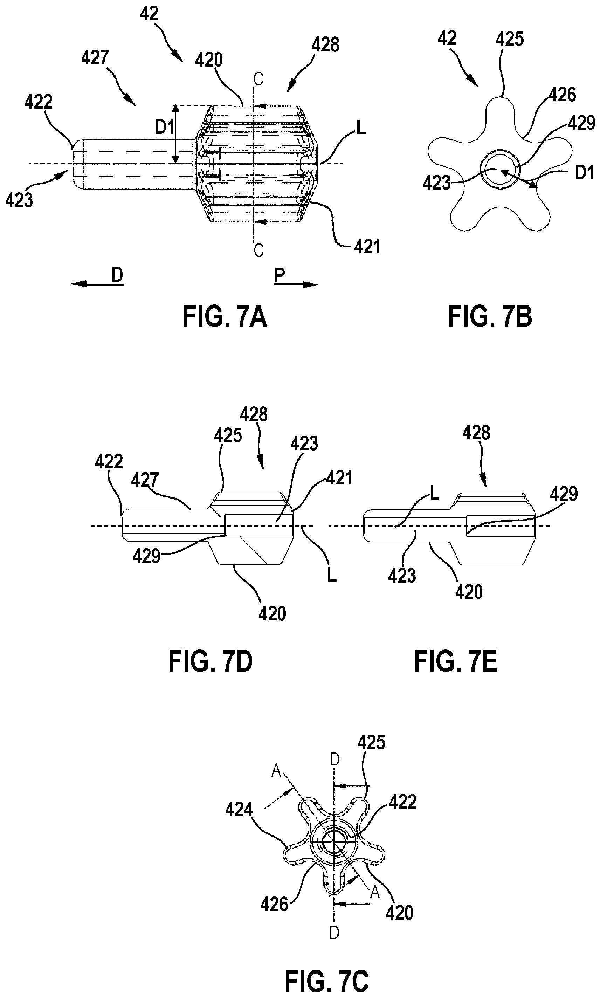

Referring to Fig. 5A to 7E three exemplary embodiments of an adapter piece are shown which may be used within the delivery system 2, wherein the adapter piece 22 shown in Fig. 5A to 5C is hereinafter referred to as first adapter piece 22, the adapter piece 32 shown in Fig. 6A to 6C is hereinafter referred to as second adapter piece 32 and the adapter piece 42 shown in Fig. 7A and 7E is hereinafter referred to as third adapter piece 42.

All three adapter pieces 22, 32, 42 comprise a lateral surface 220, 320, 420, i.e. a surface in the circumferential direction, a proximal end face 221, 321, 421 used to adjoin with the positive-locking member 212 in the first position of the adapter piece 22, 32, 42, a distal end face 222, 322, 422 and a through-hole 223, 323, 423 for receiving and fixing the tethering member 21 and the mandrel 23. Further, all adapter pieces 22, 32, 42 of Fig. 5A to 7E have chamfered edges 224, 324, 424. Additionally, each adapter piece 22, 32, 42 has a (centred) longitudinal axis L and a radial distance D1 being the distance between the respective lateral surface 220, 320, 420 and the longitudinal axis L. Therefore, dependent on the design of the adapter piece 22, 32, 42, distance D1 may vary with respect to the circumferential direction of the adapter piece 32, 42. Each of the three adapter pieces 22, 32, 42 is rotationally symmetric with respect to the respective longitudinal axis L. In addition, the through-hole 223, 323, 423 of each of the three adapter pieces 22, 32, 42 runs along the longitudinal axis L, whereby a connection of the tethering member 21 and mandrel 23 to the respective adapter piece 22, 32, 42 inside the respective through-hole 223, 323, 423 causes a centred connection and thereby prevents tilting of the respective adapter piece 22, 32, 42.

The first adapter piece 22 shown in Fig. 5A to 5C has a cylindrical shape, wherein, when the first adapter piece 22 is in its first position, the second end 212 of the tethering member 21 runs along the lateral shell surface 220 of the cylinder. Once the first adapter piece 22 is received within the inner lumen 201 of the delivery catheter 20, the inner surface of the inner lumen 201 and the lateral surface 220 of the first adapter piece 22 form one contact surface. The distance D1 of the first adapter piece 22 is formed by the radius of the cylinder and does not vary with respect to the circumferential direction of the first adapter piece 22. The cylindrical form of the first adapter piece 22 causes a very cost-effective manufacturing of the adapter piece, for example, by turning. Additionally, assembling is very simple and thereby very cost- effective because the second end 212 of the tethering member 21 may be located at any position of the circumference of the lateral surface 220. Additionally, the proximal end face 221 of the adapter piece 22 is substantially flat and runs perpendicular to the longitudinal axis L.

Fig. 6A to 6C show the second adapter piece 32 comprising a rotationally symmetrical star-shaped design with six (star) tips (if viewed in the cross-section shown in Fig. 6B) or ribs (if viewed 3-dimensionally) 325 and curved notches 326 in between two consecutive ribs 325. Accordingly, the second adapter piece 32 comprising a star shape (on its cross section) has a varying distance D1 along its circumferential direction. When the second adapter piece 32 is in its first position, i.e. received inside the inner lumen 201 of the delivery catheter 20, only the longitudinally extending ribs 325 contact the inner surface of the inner lumen 201 forming six contact lines thereby increasing trackability compared to the greater contact surface of the first adapter piece 22. Further, the curved notches 326 running parallel to the longitudinal direction L are used to guide the second end 212 of the tethering member 21 as shown in Fig. 6C. The proximal end face 321 of the adapter piece 32 is slightly curved. This helps the positive locking member 213 to slide off the second adapter piece 32 more easily, when this adapter piece 32 reaches its second position. The dimensions of the positive-locking member 213 are chosen such that it cannot pass the adapter piece 22, 32 in between the inner surface of the inner lumen 201 of the delivery catheter 20 and the lateral surface 220, 320 of the adapter piece 22, 32 in the distal direction D, while the adapter piece 22, 32 is in the first position shown in Fig. 5C and 6C with regard to the delivery catheter 20.

Referring now to Fig. 7A to 7E a third embodiment of the adapter piece 42 is shown. The third adapter piece 42 comprises a generally cylindrical tubular section 427, located at the distal end of the third adapter piece 42, and a star shaped proximal section 428 (with regard to its cross section), located at the proximal end of the third adapter piece 42 comprising five longitudinally extending ribs 425, i.e. five possible contact lines with the surface of the inner lumen 201 of the delivery catheter 20. As one can derive from Fig. 7A, D and E, the outer diameter of the tubular section 427 is smaller (e.g. between 0.2 mm and 0.4 mm) than the radial distance D1 of the third adapter piece 42, which may vary between 0.5 mm and 0.8 mm. The length of the tubular section 427 may be in between 1.5 mm and 1.7 mm. The shape and the usage of the proximal section 428 of the third adapter piece 42 is similar to the one of the second adapter piece.

The whole length of each of the three adapter pieces 22 32, 42 may be, for example, between 3 mm and 3.4 mm.

The third adapter piece 42 further has the through-hole 423 which comprises a smaller diameter along the tubular section 427 than in the proximal section 228 thereby forming an vertically extending abutting face 429 in between (see Fig. 7B, 7D and 7E). The abutting face 429 is used to limit the sliding in of the mandrel 23 during manufacturing of the delivery system prior welding. The mandrel 23 is fixed to the adapter piece 42 by welding or gluing within the through hole 423.

For attachment of the tethering member 21 to the adapter piece 42 the first end 211 of the tethering member 21 is received within the through hole 423 at the tubular section 427 of the adapter piece 42. The tubular section 427 is then crimped so that a permanent connection between the first end 211 of the tethering member 21 and the third adapter piece 42 is formed.

The materials used for the elements of the delivery system 2 are, for example, stainless steel, PEEK, ceramic

Thus, the delivery system disclosed in the present invention makes manufacturing more cost-effective as well as eases handling during implantation of a medical device, particularly through an improved construction of the adapter piece.

### List of reference numerals

- 1: Medical device (leadless pacemaker)
- 10: Housing
- 11: Electrode
- 12: Anchoring member
- 13: Connection member
- 130: Opening
- 2: Delivery system
- 20: Delivery catheter
- 200: End cap
- 201: Inner lumen
- 21: Tethering member
- 211: First end
- 212: Second end
- 213: Positive-locking member
- 22, 32 , 42: Adapter piece
- 220, 320, 420: Lateral surface
- 221, 321, 421: Proximal end face
- 222, 322, 422: Distal end face
- 223, 323, 423: Through-hole
- 224, 324, 424: Chamfered edge
- 325, 425: Rib
- 326, 426: Curved notch
- 427: Tubular section
- 428: Proximal section
- 429: Abutting face
- 23: Mandrel
- 230: Distal end
- D: Distal direction
- L: Longitudinal axis
- LA: Left atrium
- LV: Left ventricle
- M: Tissue (myocardium)
- P: Proximal direction
- RA: Right atrium
- RV: Right ventricle

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

## Claims

1. A delivery system (2) for implanting a medical device (1), comprising:
a delivery catheter (20) having an inner lumen (201),
a mandrel (23) received in the inner lumen of the delivery catheter and movable with respect to the delivery catheter,
a tethering member (21) comprising a first end (211), a second end (212) and a positive-locking member (213) fixed to the second end (212),
an adapter piece (22, 32, 42) having a longitudinal axis (L) and comprising a proximal end face (221, 321, 421) and a lateral surface (220, 320, 420), wherein the adapter piece forms a fixed connection between a distal end (231) of the mandrel and the first end of the tethering member,
wherein the adapter piece, by moving the mandrel with respect to the delivery catheter, is displaceable between a first position within the inner lumen of the delivery catheter and a second position in which the adapter piece is arranged outside of the inner lumen of the delivery catheter, wherein in the first position of the adapter piece the second end of the tethering member runs along the lateral surface of the adapter piece substantially parallel to the longitudinal axis and beyond the proximal end face of the adapter piece such that the positive-locking member adjoins the proximal end face thereby providing a locked connection with the adapter piece.

2. The delivery system of claim 1, wherein the adapter piece (22, 32, 42) is rotationally symmetric with respect to its longitudinal axis (L).

3. The delivery system of any of the preceding claims, wherein a radial distance (D1) between the longitudinal axis (L) and the lateral surface (220, 320, 420) of the adapter piece (22, 32, 42) varies along its circumferential direction.

4. The delivery system of any of the preceding claims, wherein the inner surface of the delivery catheter (20) and the adapter piece (22, 32, 42) comprise one to six contact lines or surfaces.

5. The delivery system of any of the preceding claims, wherein the adapter piece comprises a through-hole (223, 323, 423) extending along or parallel to its longitudinal axis (L) configured to receive the distal end (231) of the mandrel (23) and/or the first end (211) of the tethering member (21).

6. The delivery system of claim 5, wherein the through-hole (223, 323 ,423) comprises an abutting face (429) extending vertical to the longitudinal axis (L).

7. The delivery system of any of the preceding claims, wherein the adapter piece (42) comprises a tubular section (427) at its distal end, wherein the tubular section is configured to receive the first end (212) of the tethering member (21), and wherein the tubular section (427) has a smaller outer diameter than a proximal section (428) of the adapter piece (42).

8. The delivery system of any of the preceding claims, wherein the lateral surface (220, 320, 420) of the adapter piece (22, 32, 42) comprises at least one groove or curved notch (326, 426) extending parallel to the longitudinal axis (L).

9. The delivery system of any of the preceding claims, wherein the positive-locking member (213) of the tethering member (21) has a trapezoidal, a teardrop or a spherical shape.

10. The delivery system of any of the preceding claims, wherein the tethering member (21) forms a loop while the adapter piece (22, 32, 42) is in the first position.

11. The delivery system of any of the preceding claims, wherein dimensions of the inner lumen (201) of the delivery catheter (20), the adapter piece (22, 32, 42) and the positive-locking member (213) are chosen such that the positive-locking member is blocked from disengaging from the adapter piece in the first position but enabled to disengage from the adapter piece in the second position.

12. The delivery system of any of the preceding claims, wherein the mandrel (23) and/or the adapter piece (22, 32, 42) and/or the tethering member (21) comprises a biocompatible polymer or metal material.

13. The delivery system of any of the preceding claims, wherein a stopper (40) is arranged proximal to the adapter piece (22, 32, 42) on the mandrel (23).

14. An assembly comprising a delivery system (2) of one of the preceding claims and a medical device (1) comprising a connection member (13), wherein the tethering member (21) is coupled to the connection member while the adapter piece is in the first position.

15. The assembly of claim 14, wherein the connection member (13) comprises an opening (130) through which the loop-shaped tethering member (21) extends while the adapter piece (22, 32, 42) is in the first position.

## Patentansprüche

1. Einführsystem (2) zum Implantieren einer medizinischen Vorrichtung (1), umfassend:
einen Einführkatheter (20) mit einem Innenlumen (201),
einen Mandrin (23), der Aufnahme im Innenlumen des Einführkatheters findet und in Bezug auf den Einführkatheter beweglich ist,
ein Halteelement (21), das ein erstes Ende (211), ein zweites Ende (212) und ein formschlüssiges Element (213), das am zweiten Ende (212) befestigt ist, umfasst,
ein Adapterstück (22, 32, 42) mit einer Längsachse (L) und eine proximale Endfläche (221, 321, 421) und eine seitliche Oberfläche (220, 320, 420) umfassend, wobei das Adapterstück eine feste Verbindung zwischen einem distalen Ende (231) des Mandrins und dem ersten Ende des Halteelements bildet,
wobei das Adapterstück, indem der Mandrin in Bezug auf den Einführkatheter bewegt wird, zwischen einer ersten Position im Innenlumen des Einführkatheters und einer zweiten Position, in der das Adapterstück außerhalb des Innenlumens des Einführkatheters angeordnet ist, verschiebbar ist, wobei in der ersten Position des Adapterstücks das zweite Ende des Halteelements entlang der seitlichen Oberfläche des Adapterstücks im Wesentlichen parallel zur Längsachse und über die proximale Endfläche des Adapterstücks hinaus verläuft, sodass das formschlüssige Element an der proximalen Endfläche anliegt, wodurch eine verriegelte Verbindung mit dem Adapterstück entsteht.

2. Einführsystem nach Anspruch 1, wobei das Adapterstück (22, 32, 42) rotationssymmetrisch in Bezug auf seine Längsachse (L) ist.

3. Einführsystem nach einem der vorstehenden Ansprüche, wobei ein radialer Abstand (D1) zwischen der Längsachse (L) und der seitlichen Oberfläche (220, 320, 420) des Adapterstücks (22, 32, 42) entlang seiner umlaufenden Richtung variiert.

4. Einführsystem nach einem der vorstehenden Ansprüche, wobei die Innenfläche des Einführkatheters (20) und des Adapterstücks (22, 32, 42) eine bis sechs Kontaktlinien oder -flächen aufweist.

5. Einführsystem nach einem der vorstehenden Ansprüche, wobei das Adapterstück eine Durchgangsbohrung (223, 323, 423) umfasst, die sich entlang oder parallel zu seiner Längsachse (L) erstreckt, dafür konfiguriert, das distale Ende (231) des Mandrins (23) und/oder das erste Ende (211) des Halteelements (21) aufzunehmen.

6. Einführsystem nach Anspruch 5, wobei die Durchgangsbohrung (223, 323, 423) eine Anschlagfläche (429) umfasst, die sich vertikal zu der Längsachse (L) erstreckt.

7. Einführsystem nach einem der vorstehenden Ansprüche, wobei das Adapterteil (42) einen rohrförmigen Abschnitt (427) an seinem distalen Ende umfasst, wobei der rohrförmige Abschnitt dafür konfiguriert ist, das erste Ende (212) des Halteelements (21) aufzunehmen, und wobei der rohrförmige Abschnitt (427) einen kleineren Außendurchmesser als ein proximaler Abschnitt (428) des Adapterstücks (42) aufweist.

8. Einführsystem nach einem der vorstehenden Ansprüche, wobei die seitliche Oberfläche (220, 320, 420) des Adapterstücks (22, 32, 42) mindestens eine Rille oder runde Kerbe (326, 426) umfasst, die sich parallel zur Längsachse (L) erstreckt.

9. Einführsystem nach einem der vorstehenden Ansprüche, wobei das formschlüssige Element (213) des Halteelements (21) eine trapezförmige, tränenförmige oder kugelförmige Form hat.

10. Einführsystem nach einem der vorstehenden Ansprüche, wobei das Halteelement (21) eine Schlinge bildet, während sich das Adapterstück (22, 32, 42) in der ersten Position befindet.

11. Einführsystem nach einem der vorstehenden Ansprüche, wobei die Maße des Innenlumens (201) des Einführkatheters (20), des Adapterstücks (22, 32, 42) und des formschlüssigen Elements (213) so ausgewählt werden, dass das formschlüssige Element daran gehindert wird, sich in der ersten Position von dem Adapterstück zu lösen, es jedoch in der Lage ist, sich in der zweiten Position von dem Adapterstück zu lösen.

12. Einführsystem nach einem der vorstehenden Ansprüche, wobei der Mandrin (23) und/oder das Adapterstück (22, 32, 42) und/oder das Halteelement (21) ein biokompatibles Polymer- oder Metallmaterial umfasst.

13. Einführsystem nach einem der vorstehenden Ansprüche, wobei ein Anschlag (40) proximal zu dem Adapterstück (22, 32, 42) an dem Mandrin (23) angeordnet ist.

14. Anordnung, ein Einführsystem (2) nach einem der vorstehenden Ansprüche und ein Medizinprodukt (1) umfassend, das ein Verbindungsglied (13) umfasst, wobei das Halteelement (21) mit dem Verbindungsglied gekoppelt ist, während sich das Adapterstück in der ersten Position befindet.

15. Anordnung nach Anspruch 14, wobei das Verbindungsglied (13) eine Öffnung (130) umfasst, durch die das schlingenförmige Halteelement (21) sich erstreckt, während sich das Adapterstück (22, 32, 42) in der ersten Position befindet.

## Revendications

1. Système de libération (2) destiné à implanter un dispositif médical (1), comprenant :
un cathéter de libération (20) ayant une lumière intérieure (201),
un mandrin (23) reçu dans la lumière intérieure du cathéter de libération et déplaçable par rapport au cathéter de libération,
un élément d'attache (21) comprenant une première extrémité (211), une seconde extrémité (212) et un élément de verrouillage positif (213) fixé à la seconde extrémité (212),
une pièce d'adaptateur (22, 32, 42) ayant un axe longitudinal (L) et comprenant une face d'extrémité proximale (221, 321, 421) et une surface latérale (220, 320, 420), dans lequel la pièce d'adaptateur forme une liaison fixe entre une extrémité distale (231) du mandrin et la première extrémité de l'élément d'attache,
dans lequel la pièce d'adaptateur, en déplaçant le mandrin par rapport au cathéter de libération, peut être déplacée entre une première position au sein de la lumière intérieure du cathéter de libération et une seconde position dans laquelle la pièce d'adaptateur est agencée à l'extérieur de la lumière intérieure du cathéter de libération, dans lequel dans la première position de la pièce d'adaptateur la seconde extrémité de l'élément d'attache s'étend le long de la surface latérale de la pièce d'adaptateur de manière essentiellement parallèle à l'axe longitudinal et au-delà de la face d'extrémité proximale de la pièce d'adaptateur de telle sorte que l'élément de verrouillage positif est joint à la face d'extrémité proximale, fournissant ainsi une liaison verrouillée avec la pièce d'adaptateur.

2. Système de libération selon la revendication 1, dans lequel la pièce d'adaptateur (22, 32, 42) est symétrique en rotation par rapport à son axe longitudinal (L).

3. Système de libération selon l'une quelconque des revendications précédentes, dans lequel une distance radiale (D1) entre l'axe longitudinal (L) et la surface latérale (220, 320, 420) de la pièce d'adaptateur (22, 32, 42) varie le long de sa direction circonférentielle.

4. Système de libération selon l'une quelconque des revendications précédentes, dans lequel la surface intérieure du cathéter de libération (20) et la pièce d'adaptateur (22, 32, 42) comprennent une à six lignes ou surfaces de contact.

5. Système de libération selon l'une quelconque des revendications précédentes, dans lequel la pièce d'adaptateur comprend un orifice traversant (223, 323, 423) s'étendant le long de son axe longitudinal (L) ou parallèlement à celui-ci et conçu pour recevoir l'extrémité distale (231) du mandrin (23) et/ou la première extrémité (211) de l'élément d'attache (21).

6. Système de libération selon la revendication 5, dans lequel l'orifice traversant (223, 323, 423) comprend une face de butée (429) s'étendant verticalement à l'axe longitudinal (L).

7. Système de libération selon l'une quelconque des revendications précédentes, dans lequel la pièce d'adaptateur (42) comprend une section tubulaire (427) au niveau de son extrémité distale, dans lequel la section tubulaire est conçue pour recevoir la première extrémité (212) de l'élément d'attache (21), et dans lequel la section tubulaire (427) a un diamètre extérieur inférieur à celui d'une section proximale (428) de la pièce d'adaptateur (42).

8. Système de libération selon l'une quelconque des revendications précédentes, dans lequel la surface latérale (220, 320, 420) de la pièce d'adaptateur (22, 32, 42) comprend au moins une rainure ou encoche incurvée (326, 426) s'étendant parallèlement à l'axe longitudinal (L).

9. Système de libération selon l'une quelconque des revendications précédentes, dans lequel l'élément de verrouillage positif (213) de l'élément d'attache (21) a une forme trapézoïdale, en larme ou sphérique.

10. Système de libération selon l'une quelconque des revendications précédentes, dans lequel l'élément d'attache (21) forme une boucle tant que la pièce d'adaptateur (22, 32, 42) est dans la première position.

11. Système de libération selon l'une quelconque des revendications précédentes, dans lequel les dimensions de la lumière intérieure (201) du cathéter de libération (20), la pièce d'adaptateur (22, 32, 42) et l'élément de verrouillage positif (213) sont choisis de telle sorte que l'élément de verrouillage positif est empêché de se dégager de la pièce d'adaptateur dans la première position mais autorisé à se dégager de la pièce d'adaptateur dans la seconde position.

12. Système de libération selon l'une quelconque des revendications précédentes, dans lequel le mandrin (23) et/ou la pièce d'adaptateur (22, 32, 42) et/ou l'élément d'attache (21) comprennent un polymère biocompatible ou un matériau métallique.

13. Système de libération selon l'une quelconque des revendications précédentes, dans lequel un dispositif d'arrêt (40) est agencé en position proximale vis-à-vis de la pièce d'adaptateur (22, 32, 42) sur le mandrin (23).

14. Ensemble comprenant un système de libération (2) selon l'une des revendications précédentes et un dispositif médical (1) comprenant un élément de liaison (13), dans lequel l'élément d'attache (21) est couplé à l'élément de liaison tant que la pièce d'adaptateur est dans la première position.

15. Ensemble selon la revendication 14, dans lequel l'élément de liaison (13) comprend une ouverture (130) à travers laquelle l'élément d'attache en forme de boucle (21) s'étend tant que la pièce d'adaptateur (22, 32, 42) est dans la première position.
